# EUROPEAN PATENT SPECIFICATION

(11) **EP 4 522 144 B1**
(45) Date of publication and mention of the grant of the patent: **11.03.2026**
(21) Application number: 23726112.8
(22) Date of filing: 11.05.2023
(51) Int. Cl.: A61K 31/198, A61P 43/00, A23L 33/175, A61P 11/00

(54) **COMPOSITION COMPRISING CREATINE FOR USE IN THE TREATMENT OF BREATHING DIFFICULTIES**
ZUSAMMENSETZUNG MIT KREATIN ZUR VERWENDUNG BEI DER BEHANDLUNG VON ATEMBESCHWERDEN
COMPOSITION COMPRENANT DE LA CRÉATINE POUR UNE UTILISATION DANS LE TRAITEMENT DES DIFFICULTÉS RESPIRATOIRES

(30) Priority: 12.05.2022 EP 22172901; 28.07.2022 EP 22187487; 03.02.2023 EP 23154844; 03.02.2023 EP 23154842
(43) Date of publication of application: 19.03.2025
(73) Proprietor: Alzchem Trostberg GmbH, 83308 Trostberg (DE)
(72) Inventor: BEZLER, Jürgen, 84489 Burghausen (DE); LÖBER, Verena, 83376 Truchtlaching (DE); ALBER, Robert, 83209 Prien (DE); OSTOJIC, Sergej M., 26000 Pancevo (RS)
(74) Representative: Weickmann & Weickmann PartmbB
(86) International application number: PCT/EP2023/062670
(87) International publication number: WO 2023/217993

(56) References cited:
- STEFANO MARINARI ET AL: "Effects of nutraceutical diet integration, with coenzyme Q10 (Q-Ter multicomposite) and creatine, on dyspnea, exercise tolerance, and quality of life in COPD patients with chronic respiratory failure", MULTIDISCIPLINARY RESPIRATORY MEDICINE, BIOMED CENTRAL LTD, LONDON, UK, vol. 8, no. 1, 21 June 2013 (2013-06-21), pages 40, XP021155985, ISSN: 2049-6958, DOI: 10.1186/2049-6958-8-40
- OSTOJIC SERGEI M.: "Can creatine help in pulmonary rehabilitation after COVID-19?", THER ADV RESPIR DIS, vol. 14, 1 January 2020 (2020-01-01), pages 1 - 2, XP055971618, Retrieved from the Internet <URL:https://www.scienceopen.com/document_file/2d8f2163-41a2-4f92-9ff7-7246fd09b42d/PubMedCentral/2d8f2163-41a2-4f92-9ff7-7246fd09b42d.pdf> [retrieved on 20221016], DOI: https://doi.org/10.1177/1753466620971144

## Description

### Field of the invention:

The present invention is related to pharmaceutical compositions comprising creatine for use in the treatment of symptoms caused by a SARS-CoV-2 infection, in particular physical fatigue and breathing difficulties and/or to increase time to exhaustion,

in a subject in need thereof. A further object of the invention is a dietary supplement comprising creatine or a creatine hydrate and/or salt thereof for use in supporting the recovery from one of the conditions selected from the group of physical fatigue and breathing difficulties and/or to increase time to exhaustion, after a SARS-CoV-2 infection.

### Description of the prior art:

Although COVID-19 is seen as a disease that primarily affects the lungs, it can also damage other organs, including the heart, vascular system, kidneys and brain for example. Organ damage increases the risk of sequelae, such as cognitive impairment, heart complications (myocarditis), chronic kidney impairment, stroke, thrombosis, and Guillain-Barre syndrome.

However, most people who are infected with SARS-CoV-2 (Severe Acute Respiratory Syndrome coronavirus type 2) recover within a few weeks. But some people, even those who had mild versions of the disease, continue to experience a brought variety of symptoms after their initial recovery.

Typical symptoms that may persist after a SARS-CoV-2 infection are for example fatigue (e.g. post-viral fatigue syndrome (PVFS) or chronic fatigue syndrome (CFS)), breathing difficulties or breathlessness (dyspnea), lung or chest pain, joint pain, muscle pain or headache, concentration and memory difficulties, sleep problems (insomnia), loss of smell (anosmia) or taste (ageusia) etc., wherein fatigue, breathing difficulties (dyspnea) and chest pain belongs to the most reported conditions after acute SARS-CoV-2 infection. These symptoms sometimes persist for months in many cases.

In serious courses of disease, more than 50% of COVID-patients suffer from difficulties in breathing after 8 weeks after their discharge from the hospital as reported in a study by Manal S., Barnett J., Brill S. et al. (Thorax, 2021; 76, 396-398). These findings have been confirmed by a survey by Halpin S.J., Mclvor C., et al. (J. Med. Virol. 2021; 93, 1013-1022). More than 60% patients, who received intense medical treatment, suffered from breathing difficulties after in average 48 days after their discharge from the hospital.

The described conditions are summarized under the term "Post-COVID-19 syndrome" or "Long-COVID-19", hereinafter referred to as Post-COVID.

Subjects with breathing difficulties often get not enough air or feeling out of breath. They need to rest frequently during activity or feeling like the upper body and muscles are working harder than normal to breathe. Recommendations for these subjects are usually limited to behavioral advices, such as to allow regular rest periods, to break large tasks in smaller ones and to increase exercise slowly. The situation is very exhausting for subjects suffering under these symptoms.

People with Post-COVID often further experience muscle pain, joint pain (body pain) or chest / lung pain. Unfortunately, until to date, no specific treatments have been found for the cure of this virus. Moscatelli, F. et al., (Nutrients 2021; 13, 976-988) discuss the role of nutritional inventions and highlight the fact that strong data from clinical trials are needed to support any such assumption. Adequate nutrition is required to support the immune cell function by allowing to engage robust responses to pathogens. The micronutrients with the strongest evidence for immune support are vitamins C, D and zinc.

Practical medical treatment strategies of patients suffering from Long-COVID are summarized in Deutsches Ärzteblatt 2020; 49, 117 and are based on primary care recommendations of Long-Covid as described by Greenhalgh, T.; Knight M., A'Court C.; BMJ 2020; 370, 3026. Medical management is limited to symptomatic, such as treating fever with paracetamol and considering antibiotics for secondary infection.

To improve the recovery of patients with breathing difficulties or lung pain, pulmonary rehabilitation treatment should be applied as soon as possible. Pulmonary rehabilitation is regarded as one of the most important interventions in the treatment of Post-COVID, so far.

Suitable pulmonary rehabilitation measures are described in Wang T. J. et al. (Am. J. Phys. Med. Rehabil., 2020 Sep; 99(9), 769-774).

Creatine is a methylguanidinoacetic acid usually available from animal-based foods and/or produced naturally in the body from the amino acids arginine, glycine and methionine. Endogenous creatine synthesis provides about half of the daily need. The remaining amount of creatine needed to maintain normal tissue levels of creatine is obtained in the diet from animal based products such as fish or meat or from dietary supplements. Creatine plays a vital role in the energy metabolism in every cell of the body. It mainly serves as a metabolic intermediary of energy transfer by facilitating the recycling of ATP, the source of energy for use and storage at the cellular level. Thus, creatine is found in high concentrations in organs with high energy turnover, with ~95% of the human body's creatine stores in the skeletal muscle and the remaining 5% in the brain, liver, kidney, and testes (McCall, W., Persky, AM. Pharmacokinetics of creatine. Subcell Biochem 2007, 46, 261-273; Bonilla, D.A. et al., Metabolic Basis of Creatine in Health and Disease: A Bioinformatics-Assisted Review. Nutrients 2021, 13, 1238; Brosnan, M.E. et al., The role of dietary creatine. Amino Acids 2016, 48, 1785-1791; Harris, R. Creatine in health, medicine and sport: An introduction to a meeting held at Downing College, University of Cambridge, July 2010, Amino Acids 2011, 40, 1267; Harris, R.C.et al. Elevation of creatine in resting and exercised muscle of normal subjects by creatine supplementation. Clin. Sci. 1992, 83, 367-374; Kreider, R.B.; Stout, J.R. Creatine in Health and Disease. Nutrients 2021, 13, 447; Ostojic, S.M.; Forbes, S.C. Perspective: Creatine, a Conditionally Essential Nutrient: Building the Case. Adv. Nutr. 2021, 00, 1-4).

The administration of creatine has therefore been considered as a supportive measure for the treatment of a variety of diseases. In particular Ostojic, S.M. et al. for example describes a dietary treatment of chronical fatigue syndrome (CFS) comprising guanidinoacetic acid (GAA) (Nutrients 2016, 8, 72). The administration of a combination of creatine and coenzyme Q10 for the treatment of COPD patients is described in Marinari, S. et al., Multidisciplinary Respiratory Medicine 2013, 8:40. The potency of creatine in the treatment of post-viral fatigue syndrome (PVFS) is discussed in Ostojic, S.M. Nutrients 2021, 13, 503; Ostojic, S.M., Therapeutic Advances in Respiratory Disease, 2020, Vol. 14, 1-2 and in Kreider R.B. et al., Nutrients 2021, 13, 447.

Starting from this the problem to be solved by the present invention is to improve the recovery from physical fatigue and breathing difficulties and/or to increase time to exhaustion caused by SARS-CoV-2 infection, particularly when these conditions are symptoms of post viral fatigue syndrome (PVFS).

### Description of the invention:

The problem is solved by administration of creatine in patients in need thereof. The present invention relates to a pharmaceutical composition comprising creatine or a creatine hydrate and/or a salt thereof for use in the treatment of one of the conditions selected from group of physical fatigue and breathing difficulties and/or to increase time to exhaustion, after a SARS-CoV-2 infection.

The present invention further relates to a dietary supplement comprising creatine or a creatine hydrate and/or salt thereof for use in supporting the recovery from one of the conditions selected from the group of physical fatigue and breathing difficulties and/or to increase time to exhaustion, after a SARS-CoV-2 infection.

Creatine supports the recovery from physical fatigue and/or breathing difficulties caused by a SARS-CoV-2 infection, and in particular supports the recovery from physical fatigue and/or breathing difficulties after a SARS-CoV-2 infection, e.g. Post-Covid.

The administration of creatine is particularly useful for patients suffering from physical fatigue and/or breathing difficulties after a SARS-CoV-2 infection, e.g. Post-Covid in combination with pulmonary rehabilitation measures, in particular with breathing exercises and/or with physical exercises. By combination of pulmonary rehabilitation measures, such as training of the respiratory musculature by breathing exercises, and simultaneous administration of creatine, the recovery of patients, in particular of Post-COVID patients, suffering under breathing difficulties is significantly improved, compared to the ingestion of creatine or the application of breathing exercises alone.

Post-viral fatigue syndrome (PVFS) is a perplexing long-term neurological disorder. PVFS is in particular characterized by an inability to participate in routine activities that were possible before becoming ill, lasting for more than six months and accompanied by fatigue, post-exertional malaise and unrefreshing sleep. PVFS-related symptoms are in particular often found after infection with a member of the corona virus family (SARS-CoV2) leading in many cases to Post-COVID fatigue syndrome.

Therefore, a first embodiment of the invention is a pharmaceutical composition comprising creatine or a creatine hydrate and/or a salt thereof for use in the treatment of physical fatigue and/or breathing difficulties and/or to increase time to exhaustion, after a SARS-CoV-2 infection, in particular after a COVID-19 infection. In a preferred embodiment of the invention the pharmaceutical composition comprising creatine or a creatine hydrate and/or a salt thereof is used for the treatment of breathing difficulties in combination with breathing exercises and/ or with physical exercises.

A second embodiment of the invention is a dietary supplement comprising creatine or a creatine hydrate and/or salt thereof for use in supporting the recovery from one of the conditions selected from the group of physical fatigue and breathing difficulties and/or to increase time to exhaustion, after a SARS-CoV-2 infection, in particular after a viral infection of the lungs or lower respiratory system, preferably in combination with physical exercises and/or breathing exercises.

By the administration of creatine a significant increase of the creatine concentration in muscles and in brain of Post-COVID patients can be detected.

This is surprising, since it was assumed previously that creatine cannot cross the blood-brain barrier. Within the framework of the present invention it has been found, however, that seemingly long-COVID or Post-COVID causes changes in the blood-brain barrier so that a creatine uptake and enrichment in brain areas such as the thalamus, grey matter and white matter, specifically in post-COVID situations was observed.

Further, the inventors of the present application found that Long-COVID patients, without supplementation of creatine, show only low levels of creatine in the brain. Within the framework of the present invention it was found that the concentrations of total creatine in the brain, and in particular in the thalamus, in the white matter and in the grey is significantly decreased in Long-COVID patients compared to the reference values of the general population. Thus, without being bound to any theory, it is assumed that one of the effects and/or causes of Long-COVID is depletion of creatine in brain areas and, thus, decrease of the creatine level in such brain areas. By the inventive finding that creatine is able to cross the blood-brain barrier in Long-COVID patients the creatine levels in the brain areas can be enriched and/or increased by the addition of creatine.

It is particular surprising that while the concentration of total creatine in the brain was found to be reduced in Long-COVID patients compared to reference values for the general population on the one hand, on the other hand, a significant enrichment of creatine in the brain, in particular in the thalamus, grey matter and/or white matter was found in Post-COVID patients, while no or only very little increase of creatine in the brain of about max. 5% is seen in normal healthy population after creatine supplementation. Seemingly, Long-COVID effects a depletion of brain creatine and at the same time alters the properties of the blood-brain barrier, so that supplemented creatine can pass, and results in a significant increase in creatine in the brain after creatine supplementation. Surprisingly, according to the invention an enrichment of creatine in the brain after creatine supplementation was found indicating that creatine in Long-COVID patients can pass the blood-brain barrier. It was in particular found that supplementation of creatine alone, i.e. supplementation of creatine without any transporters or auxiliary agents known for altering the blood-brain barrier results in enrichment of creatine in the brain.

A significant increase of the creatine concentration in muscles and in brain of Post-COVID patients is particularly surprising for patients treated with physical exercise and/or breathing exercises, because tissue total creatine concentrations remained largely unresponsive to physical exercise and/or breathing exercise (or even further diminished from baseline levels), suggesting long-term disturbances in tissue bioenergetics in Post-COVID. Thus, the recovery from Post-COVID is supported by the administration of creatine to improve tissue creatine levels, e.g. in muscles and across the brain. Additionally the physical situation of Post-COVID patients, such as reduced physical fatigue, reduced breathing difficulties, reduced lung (chest) pain und reduced lung malaise can be improved. Also the time to exhaustion is increased for subjects receiving creatine in combination with physical exercises and/or breathing exercises. So the physical situation of Post-COVID patients can be improved significantly by the combination of creatine administration and physical exercises and/or breathing exercises.

Creatine effects could be augmented if individuals take part in exercise during supplementation. Breathing exercise could induce hyperemia that increases tissue perfusion, enhancing creatine delivery to the target cells (Ribeiro F, Longobardi I, Perim P, Duarte B, Ferreira P, Gualano B, Roschel H, Saunders B. Timing of creatine supplementation around exercise: a real concern? Nutrients. 2021;13(8):2844). Exercise potentiates the resultant increase in intramuscular creatine during creatine intake, with greater increases in the exercised versus non-exercised body segments (Robinson TM, Sewell DA, Hultman E, Greenhaff PL. Role of submaximal exercise in promoting creatine and glycogen accumulation in human skeletal muscle. J Appl Physiol. 1999;87(2):598-604). In addition, exercise could upregulate sodium-potassium pump that activates specific creatine transporter (CT1) and enable more creatine delivered to the specific cell (Odoom J.E., Kemp G.J., Radda G.K. The regulation of total creatine content in a myoblast cell line. Mol. Cell. Biochem. 1996;158:179-188). It is assumed that, without bound to theory, these effects support not only the recovery of Post-COVID patients by creatine administration, it minimizes undesired effects (e.g. increased physical fatigue) of physical exercise of the patients, including breathing exercise, in addition. Evidence from studies suggest that exercise enhances creatine accrual in target tissues with creatine supplementation, implying a synergistic effect of creatine, in particular of creatine monohydrate plus breathing exercise.

The pharmaceutical composition of the first embodiment and the composition used as dietary supplement of the second embodiment are referred to herein after also as creatine composition, creatine comprising composition or composition comprising creatine. The terms "creatine composition", "creatine comprising composition" or "composition comprising creatine" comprise creatine or a creatine hydrate and/or a salt thereof if not expressly stated otherwise.

The creatine comprising compositions of the present invention are particularly useful to support recovery from typical symptoms after virus infection, such as breathing difficulties. Breathing difficulties or dyspnea are characterized by shortness of breath, and impaired ability to inhale or exhale. Breathing difficulties can happen as a consequence of several acute and chronic cardiopulmonary conditions, including asthma, chronic obstructive pulmonary disease (COPD), heart diseases, pneumonia etc. In such cases breathing difficulties are a respiratory disease, while Post-Covid is classified as a neurological disease. In COPD as a respiratory disease, for example, oxidative stress plays a key role and therefor treatments thereof often include antioxidants such as Coenzyme Q10. However, breathlessness is one of the most occurring conditions after virus infection, in particular after corona virus infections including infections with SARS-CoV-2. In such cases breathlessness or breathing difficulties associated with a virus infection and in particular with a infection with SARS-CoV-2 often persists for weeks or months in patients with an overcome virus infection. Thus the cause and mechanism underlying a classical COPD being a respiratory disease and breathing difficulties associated with Post-Covid, being a neurological disease are seemingly different. Breathing difficulties can be further associated with low blood oxygen levels that could observed over longer periods of time and compromise general health.

Chest pain (lung pain) is a sharp throbing sensation that happens during breathing, coughing or sneezing. The most common cases of chest pain or lung pain are asthma, chronic obstructive pulmonary disease, and in particular bacterial or viral infections of pleura (pleuritis) or other thoracal tissues. Chest (lung) pain is for example observed as persistent condition after virus infection such as SARS-CoV-2 infection. Chest (lung) pain could be long lasting or chronic, and is often accompanied by cough, breathing difficulties, and wheezing. When pleuritic inflammation occurs near the diaphragm, pain can be referred also to the neck or shoulder.

Several acute and chronic viral infections which cause breathing difficulties and/or lung malaise, chest (lung) pain, include influenza A or B viruses (e.g., H1N1, H5N1), enteroviruses, respiratory syncytial virus, parainfluenza, adenovirus, and coronaviruses (e.g. SARS, MERS, SARS-CoV-2). The conditions can persist for weeks or months. After a SARS-CoV-2 infection, for example, breathlessness can persist from 2 weeks up to 1 year or even longer.

Breathing difficulties within the meaning of the invention comprises particularly breathlessness (dyspnea) caused by virus infection. Chest pain includes particularly pleuritic and thoracal chest pain (lung pain) caused by viral infection.

According to the present invention the pharmaceutical composition comprising creatine or a creatine hydrate and/or a salt thereof is in particular for use in the treatment of one of the conditions selected from group of physical fatigue and breathing difficulties and/or to increase time to exhaustion in Post-COVID patients. Herein in particular symptoms and/or conditions which persist or occur after initial recovery from acute COVID infection are designated Post-COVID. One of the difficulties associated with finding suitable treatments of PVFS and in particular of Post-COVID is the variety, multiplicity, diversity and vagueness of symptoms associated with Post-COVID and at the same time the uncertainty about the causes of the symptoms. This complicates both the treatment of Post-COVID and a forecast of which agents or treatments might function in the treatment of Post-COVID. Manifestations of Post-COVID occur as many different conditions and symptoms including pulmonary conditions, neurological symptoms and conditions such as headaches, nasal smell disorders, impaired sense of taste, dizziness, mental confusion, disorientation, and other impairments, neuropsychiatric disorders, strokes, gastrointestinal symptoms such as nausea, loss of appetite, vomiting, diarrhea; cardiovascular diseases including myocarditis, cardiac insufficiency, cardiac failure, and thromboembolic events; rhenal insufficiency; dermatological manifestations. In particular with regard to long-term effects, no uniform clinical picture can be defined, and the underlying mechanisms are not clear. Post-COVID patients report quite different symptoms which persist over weeks and even over months. Quite often reported complaints or symptoms include fatigue, weariness, mental fatigue, exhaustion, impaired resilience, memory problems, sleep problems, muscle weakness, muscle pain and psychic problems such as depressive symptoms and anxiety. Other symptoms reported include deterioration of pulmonary function, deterioration of lung function, deterioration of kidney function and heart muscle inflammation. This list is in no way conclusive, however, shows the diversity and variety of conditions and symptoms associated with Post-COVID. What is even more unknown up to date are the causes initiated by COVID or Post-COVID for those conditions and symptoms. The provision of suitable treatments of Post-COVID is therefore difficult, since mutual applicability of treatments known for similar symptoms does not exist.

According to the present invention it was now surprisingly found that a pharmaceutical composition comprising creatine or a creatine hydrate and/or a salt thereof is effective for the treatment of specific conditions selected from the group of physical fatigue and breathing difficulties and/or to increase time to exhaustion associated with Post-COVID. As outlined, the causes of the numerous various symptoms associated with Post-COVID are not known and, thus, an effective treatment is not predictable. Further, it appears that not only the symptoms are quite numerous but also the conditions causing the various symptoms. In the tests and experiments underlying the present invention it was now surprisingly found that provision of creatine specifically improves the status relating to physical fatigue and breathing difficulties of Post-Covid patients.

The recovery of patients suffering under breathing difficulties after a viral infection can be surprisingly improved by administration of creatine, in particular in combination with breathing exercises. Creatine is also known as methylguanidinoacetic acid, which is naturally occurring in the body of animals and humans. Other names for creatine are *N*-(Aminoiminiomethyl)-*N*-methyl-glycine or *N-*Methyl-N-guanylglycine. Creatine is further available from animal-based foods or in higher amounts as food supplement. In food supplements preferably creatine monohydrate is used, which can be prepared in very high purity.

Beside creatine, also a creatine hydrate and/or a salt thereof can be used in accordance with the invention. Creatine derivatives can be natural occurring compounds, such as creatine phosphate, or pro-drugs of creatine, which are able to release creatine under physiological conditions, such as creatine esters. In the context of the present invention, guanidino acetic acid (GAA) is also included in the group of creatine derivatives. Physiologically acceptable creatine derivatives are preferably selected from the group consisting of creatine, creatine hydrates, creatine esters or amides, including creatine C₁-C₅-alkyl esters, N-C₁-C₅-alkyl amides, creatine phosphate, creatinol-O-phosphate or a mixture thereof.

Creatine salts, creatine adducts, salts of physiologically acceptable creatine derivatives and adducts of the physiologically acceptable creatine derivatives are preferably selected from the group consisting of the corresponding acetates, citrates, maleates, fumarates, tartrates, malates, pyruvates, ascorbates, succinates, aspartates, lactates, oxalates, formates, benzoates, phosphates, sulfates, chlorides, hydrochlorides, of the corresponding potassium, sodium, calcium, magnesium salts, of the corresponding L-carnitine, acetyl-L-carnitine, taurine, betaine, choline, methionine adducts or a mixture thereof.

As used herein the term breathing exercises includes physical exercises and also includes pulmonary rehabilitation.

Pulmonary rehabilitation is a component of the management of people with respiratory diseases or lung problems due to other conditions. Pulmonary rehabilitation includes exercise training, health education, and breathing techniques aimed to improve decreased pulmonary function and improve symptoms of dyspnea. Breathing exercises are recommended during the acute management of various pulmonary diseases, including COVID-19 (Wang, T.J. et al., PM&R and Pulmonary Rehabilitation for COVID-19, Am J Phys Med Rehab, 2020 Sep;99(9):769-774. doi: 10.1097/PHM.0000000000001505).

Pulmonary rehabilitation includes training of respiratory muscles and of muscles of exhalation by breathing exercises. Respiratory muscles comprise inspiratory muscles such as the diaphragm and the external intercostal muscles, e.g. musculi intercostales externi and musculi intercartilaginei, which are attached between the ribs. Diaphragm and external intercostal muscles are one of the most important groups of respiratory muscles. Further inspiratory muscles are summarized as accessory muscles of inspiration. This group of muscles support the inspiration through the lungs and comprise the, musculus serratus posterior superior, musculus serratus posterior inferior, musculus pectoralis minor et major, musculus sternocleidomastoideus, and musculus erector spinae.

The muscles of exhalation comprises internal intercostal muscles (musculi intercostales interni et intimi), lower rip muscles (musculi subcostales) and accessory exhalation muscles, such as musculus obliquus internus abdominis, musculus transversus abdominis, musculus transversus thoracis, musculus latissimus dorsi, musculus qudratus lumborum, and musculus rectus abdominis.

Treatment with creatine can already begin during the virus infection, preferably within about three months (12 weeks) after infection. Preferably creatine administration starts in a time frame from 2 weeks after infection to 8 weeks after infection. Furthermore, the creatine administration should start most preferably within 4 weeks before physical exercises and/ or breathing exercises including pulmonary rehabilitation begins or concurrently with physical exercises and/ or breathing exercises including pulmonary rehabilitation. However, administration of creatine after the physical exercises and/ or breathing exercises including pulmonary rehabilitation have started is also possible.

Pulmonary rehabilitation measures, such as training of the respiratory musculature by breathing exercises should be started as soon as possible, provided that the patient's state of health permits this. The rehabilitation, physical exercises and/ or breathing exercises are usually started within 20 weeks, preferably within 12 weeks, most preferably within 6 weeks, after infection has subsided.

The duration of supplementation of creatine lasts normally between 1 week and 12 months or longer, preferably between 1 and 8 months, in particular between 3 and 6 months depending on the symptoms of the subject in need thereof.

The amount of creatine to be administered is in the range of 3g to 30g per day. Preferably the dosage is in the range of 7g to 25g, most preferred between 8g and 20g, which is higher than commonly recommended for sportsmen or sportswomen.

Preferably the administration of creatine is divided in an accumulation phase and a maintaining phase, wherein the daily dose of creatine in the composition is in the range of 10g to 30g in the initial accumulation phase and in the range of 7g to 15g for the subsequent maintaining phase. The accumulation phase has a duration of up to 3 weeks, preferably between 3 and 14 days, particularly between 5 and 10 days and the maintaining phase has a duration of 1 week to 12 months, preferably between 2 and 8 months, in particular between 3 and 6 months.

The accumulation phase is usually the initial phase. However, additional accumulation phases, for example with a duration between 1 day and 7 days, may be integrated into the maintaining phase.

The daily creatine dose can be administered once a day, during breakfast for example, or spread over 2, 3, 4 or 5 times a day.

If the administered creatine composition comprises a creatine derivative, pro drug, adduct or salt, the amount of the creatine moiety contained therein is decisive for the daily dosage within the ranges described above.

The creatine composition described herein may be preferably administered orally, in form of tablets, coated tablets, capsules, granules or powders.

In particular, granulates or powders comprising the creatine composition are used as aqueous suspension or in water-soluble form. The solubility of pure creatine and several creatine derivatives is low. Creatine for example has a solubility of 17 g/L (at 20°C). Creatine and derivatives thereof with low solubility can be used in form of an aqueous suspension. A disadvantage of an aqueous suspension is often, that it segregates before ingestion. Therefore water-soluble granules or powders are usually preferred. To increase the water solubility of creatine or creatine derivatives water soluble salts or adducts thereof may be used. To increase the water-solubility particularly the use of acids or complexing agents may be useful to provide the corresponding creatine salts or creatine derivative salts. Examples for suitable acids, including carboxylic acids, and complexing agents are selected from the group malic acid, aspartic acid, ascorbic acid, succinic acid, pyruvic acid, fumaric acid, gluconic acid, alpha-ketoglutaric acid, oxalic acid, acetic acid, formic acid, sulfuric acid, hydrochloric acid, L-carnitine, acetyl-L-carnitine, taurine, betaine, choline, and lipoic acid. Peptides and amino acids may be also useful to increase the solubility of creatine and creatine derivatives. Further sodium, potassium, calcium and magnesium salts may be used to increase the water-solubility of creatine or creatine derivatives.

The molar ratio of creatine or the creatine derivatives and the acids or complexing agents mentioned above is usually in the range of 5:1 to 1:5, preferably in the range of 2:1 to 1:2, in particular in the range of 1.3:1 to 1:1.3.

The granules and powders can also be used for preparing foodstuff supporting the recovery from physical fatigue, breathing difficulties, breathlessness, chest / lung pain, lung malaise, and body ache after a viral infection, in particular in Post-COVID patients.

The creatine composition used in accordance with the present invention may be applied further in tablet form.

The creatine composition may be tableted as such or in form of a formulation comprising excipients. Suitable excipients are for example pharmacologically inactive ingredients, such as binders, fillers, antioxidants, preservatives, stabilizers, anti-caking agents, lubricants, disintegrants, flavors, pigments etc.

As binder or filler a wide variety of compounds may be used. Dibasic calcium phosphate; saccharides, including lactose and sucrose; polysaccharides and derivatives thereof, including starches, cellulose, modified cellulose, and cellulose ethers, such as hydroxypropyl cellulose or hydroxyethyl cellulose; microcrystalline cellulose; sugar alcohols, such as xylitol, sorbitol or mannitol; peptides, such as gelatin; polymers, e.g. polyvinylpyrrolidone, polyethylene glycol, etc. are common binders or fillers for tablets.

Typical suitable preservatives are for example cysteine, methionine, citric acid, sodium citrate, tatrazines or synthetic preservatives like parabens, including methyl paraben and propyl paraben, and benzoic acid. Suitable antioxidants may be selected from group of vitamin A, vitamin C, vitamin E, retinyl palmitate, and selenium.

Lubricants and anti-caking agents reduce the adhesion in granulates or powders and thus prevent sticking to tablet punches. They are also used to help protect tablets from sticking. The most commonly used anti-caking agents is magnesium stearate, stearic acid, or stearin, but also other magnesium or calcium salts of fatty acids may be used instead or in addition. Common mineral lubricants for example are talc or silica.

Disintegrants expand and dissolve when wet causing a tablet to break apart in the digestive tract, or in specific segments of the digestion process, releasing the ingredients for absorption by the body. Examples of disintegrants include crosslinked polymers like crosslinked polyvinylpyrrolidone (crospovidone) and crosslinked sodium carboxymethyl cellulose (croscarmellose sodium). Other suitable disintegrants are for example modified starch or sodium starch glycolate.

Flavors can be used to mask unpleasant tasting tablet ingredients. Further the ingredients may increase the patients' acceptance of the tablets. Flavorings may be natural, e.g. fruit extracts, or artificial. Natural extracts of vanilla, peach, apricot, raspberry, mint, anise or cherry can be used as flavors, for example. Antacid compounds or cough syrup would be also suitable.

Suitable pigments and colours are for example food colourants.

Tablet coatings protect tablet ingredients from deterioration by moisture in the air and make large or unpleasant-tasting tablets easier to swallow. For most coated tablets, a cellulose ether, particularly hydroxypropyl methylcellulose (HPMC) film coating is used. But other coating materials are also useful, for example synthetic polymers, shellac, plant fibers, waxes, fatty acids or polysaccharides. Capsules are coated usually with gelatine.

Particularly useful coatings for tablets related to the invention disclosed herein are crosslinked sodium carboxymethyl cellulose (croscarmellose sodium), silicified microcrystalline cellulose, agglomerated anhydrous lactose and lactitol monohydrate.

Preferred tablets comprise at least
a) 10 wt.-% to 100 wt.-%, preferably 30 wt.-% to 99 wt.-% creatine, creatine hydrate or salts thereof;
b) 0 to 80 wt.-% carbohydrates;
c) 0 to 20 wt.-% anti-caking agents selected from fatty acids or fatty acid salts, particularly magnesium stearate;
d) 0 to 20 wt.-% acidifier, preferably citric acid;
e) 0 to 20 wt.-% fatty acids, preferably coconut oil;
f) 0 to 5 wt.-% flavors.

Preferred capsules comprise at least
a) 10 wt.-% to 95 wt.-%, preferably 30 wt.-% to 80 wt.-% creatine, creatine hydrate or salts thereof
b) 0 to 80 wt.-% carbohydrates;
c) 0 to 20 wt.-% anti-caking agents selected from fatty acids or fatty acid salts, particularly magnesium stearate,
d) 0 to 5 wt.-% flavors;
e) 5 to 90 wt.-% gelatin.

The described ingredients may be useful not only in tablets but also in granules or powders comprising creatine or creatine derivatives or salts or adducts thereof. Particularly binders, fillers, antioxidants, preservatives, stabilizers, anti-caking agents, lubricants, disintegrants, flavors, and pigments can be part of the creatine composition used in accordance with the present invention.

Preferred granules or powders relating to the described invention comprise 10 wt.-% to 100 wt.-%, preferably 30 wt.-% to 99 wt.-% creatine, creatine derivatives or salts or adducts thereof.

In combination with creatine, creatine hydrate or salts thereof, anti-inflammatory drugs can be applied to support the recovery from breathing difficulties (dyspnea) and/or chest pain, lung pain, lung malaise, and body ache caused by virus infections. Anti-inflammatory drugs are for example non-steroidal drugs, such as aspirin, ibuprofen, naproxen, diclofenac, celecoxib, mefenamic acid, etoricoxib, indomethacin etc. or steroids, such as corticosteroids, including cortisone, hydrocortisone and prednisone.

Further combinations of creatine, creatine hydrate or salts thereof, with neuroprotective drugs can be advantageous. Preferred neuroprotective agents include glutamate excitotoxicity inhibitors such as ginsenoside, riluzole, progesterone, estrogen, memantine, or simvastatin; stimulants such as caffeine; growth factors such as IGF-1, CNTF; nitric oxide synthase inhibitors; and caspase inhibitors or erythropoietin.

A combination of creatine, creatine hydrate or salts thereof, with drugs for the treatment of the virus infection is also possible, in particular if the administration of creatine is started during the acute phase of the virus infection.

For the treatment of COVID-19 drugs of the group Lagevrio (molnupiravir), Olumiant (baricitinib), (tixagevimab / cilgavimab), Kineret (anakinra), Paxlovid (PF-07321332 / ritonavir), Regkirona (regdanvimab), RoActemra (tocilizumab), Ronapreve (casirivimab / imdevimab), Veklury (remdesivir), Xevudy (sotrovimab) are available for example. For the treatment of influenza Rapivab (peramivir), Relenza (zanamivir), Tamiflu (oseltamivir phosphate), Xofluza (baloxavir marboxil) are suitable drugs. For the treatment of adenoviruses cidofovir, ribavirin, ganciclovir, and vidarabine, are useful drugs.

Creatine, creatine hydrate or salts thereof can be administered in combination with creatine precursors, such as guanidinoacetate / guanidinoacetic acid, and/or methyl group donors, such as arginine, glycine, methionine etc. or other compounds or precursor useful in the energy pathway of cells. However, in a preferred embodiment creatine, creatine hydrate or salts thereof are applied without additional administration of such compounds, particularly without co-administration of ubiquinone 10 (coenzyme Q-10).

Creatine, creatine hydrate or salts thereof are preferably used in combination with a pain-reducing, anti-inflammatory diet according to a preferred embodiment of the present invention. The preferred diet shall be provide all nutrients for normal energy metabolism and healthy function of the nervous system. A particularly suitable diet should comprise vitamins, minerals, unsaturated fatty acids, amino acids, secondary plant metabolites, including anti-oxidative agents, phytonutrients or essential or semi-essential nutrients.

For a pain-reducing, anti-inflammatory diet particularly vitamins of the group of vitamin, C, vitamin D, vitamin E, vitamin K and B vitamins (thiamine, riboflavin, nicotinamide, pantothenic acid, pyridoxine, biotin, folate, and/or vitamin B₁₂) should applied in sufficient amounts.

Important minerals are selected from group of magnesium, calcium, potassium, sodium, copper, manganese, zinc, selenium, and boric acid / boron.

The vitamins and minerals should be present in the diet in sufficient quantities so that no deficiency symptoms occur. Appropriate recommendations for the daily amount of these nutrients are issued by the German Society for Nutrition (Deutsche Gesellschaft für Ernährung e.V., Referenzwerte für die Nährstoffzufuhr, 2. Auflage, 7. Aktualisierte Ausgabe, 2021).

A further group of useful nutrients for a pain-reducing, anti-inflammatory diet are unsaturated fatty acids, in particular omega-3 fatty acid; docosahexanoic acid (DHA), eicosapentenoic acid (EPA), alpha-lipoic acid and lecithine.

A preferred diet should be also rich on amino acids selected from the group of L-tyrosine, arginine and glycine. Additional amino acids for example theanine, cystine, taurine, or mixtures thereof are enriched in a preferred diet.

Further compounds that should be present in a pain-reducing, anti-inflammatory diet are N-acetyl-L-cysteine, gamma-amino-butyric acid (GABA), S-adenosylmethionine (SAMe), ubiquintol (coenzyme Q10), NADH, resveratrol, lutein, lycopene, choline, and carnitine.

Phytonutrients, including secondary plant metabolites, which are particularly useful in a pain-reducing, anti-inflammatory diet are for example antioxidants, anthocyanidines, flavonoids, flavones, isoflavones, catechins, anthocyanidins, isothiocyanates, carotenoids, allyl sulfides, polyphenols, resveratrol, lutein, or lycopene. Particularly preferred secondary plant metabolites are oligomeric procyanidins and oligomeric proanthocyanidins (OPCs).

For a preferred diet also plants, particularly herbs, spices, fruits, vegetables and legumes, or extracts, oils, powders, or compounds thereof can be used, for example from the group boswellia serrata, curcuma longa, grape seeds (in particular with OPCs), or devil's claw. Further tomatoes, olive oil, green leafy vegetables, such as spinach, kale, and collards, nuts like almonds and walnuts, or fruits such as strawberries, blueberries, cherries, and oranges are regarded as anti-inflammatory foods. Fatty fish like salmon, mackerel, tuna, and sardines have also anti-inflammatory effects.

The recommended nutrients for a pain-reducing, anti-inflammatory diet can be supplied by selecting suitable foods or by addition of the respective components via nutrition supplements.

### Description of the figures:

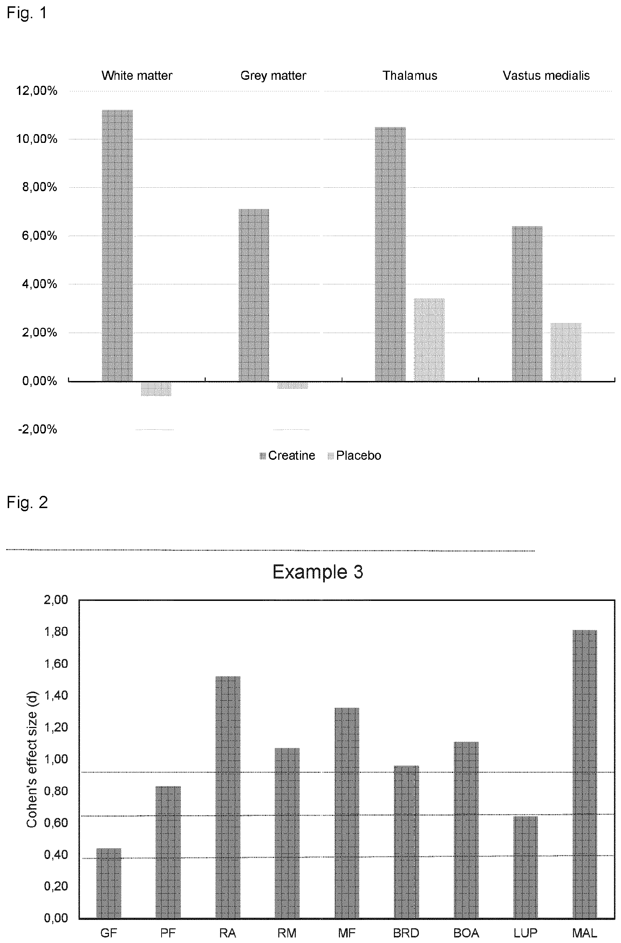

Figure 1: Changes in tissue creatine levels in patients suffering under Post-COVID. Left columns representing the group of patients receiving creatine, right columns representing the placebo group. Figure 2 depicts calculated Cohen's effect sizes of the administration of creatine in Post-COVID patients (GF, general fatigue; PF, physical fatigue; RM, reduced motivation; RA, reduced activity; MF, mental fatigue; BOA, body aches; BRD, breathing difficulties; LUP, lung pain; and MAL, malaise).

### Working examples:

### Example 1:

A study was conducted to evaluate effectiveness and safety of replenishing creatine via dietary supplementation in patients suffering under Post-COVID after a SARS-CoV-2 infection. This study employed a parallel-group, randomized placebo-controlled double-blind design. The allocation ratio to experimental group (creatine) and control group (placebo) was set at 1:1. The eligibility criteria for patients to be included in the trial were: age 18-65 years, COVID-19 positive test within last 3 months (as documented by valid PCR or antigen test) moderate-to-severe fatigue and at least one of additional COVID-19 related symptoms selected from the group of anosmia, ageusia, breathing difficulties, lung pain, body aches, headaches, and concentration difficulties. Exclusion criteria were other pulmonary and cardiovascular conditions, and history of dietary supplement use during the 4 weeks before the trial commences.

The study was conducted in compliance with the Declaration of Helsinki (7th revision). The data presented until now were collected at FSPE Applied Bioenergetics Lab at the University of Novi Sad, from October 2021 to January 2022. The experimental (creatine) group received 4 grams of creatine monohydrate per day, while control group (placebo) received equivalent amount of inulin. The participants were asked to take the intervention one time per day during breakfast, with experimental or control powder stirred in 250 mL of lukewarm water and consumed immediately afterwards. Both interventions were similar in appearance, texture, and sensory characteristics. Creatine monohydrate was provided by Alzchem Trostberg GmbH (Trostberg, Germany). The duration of intervention was 6 months, and participants were asked to refrain from using any other dietary supplements during the trial. All outcome measures were determined at baseline (pre-administration) and after 3 months and 6-months. The primary outcome was the change in vastus medialis creatine levels and the creatine levels in brain at baseline and at 6-month follow-up. The minimal sample size (n = 12) was calculated using power analysis (G*Power 3.1.9.3, Heinrich-Heine-Universität Düsseldorf), with the effects size set at 0.50 (medium effect), alpha error probability 0.05, power 0.80 for two groups, and two measurements (3 months) and three measurements (6-months follow-up), respectively, of study outcomes.

The number of participants who were randomly assigned, received intended treatment, and were analyzed for the primary outcome at this time was twelve in total, six in experimental group and six in control group. The participants recruited reported no major side effects of either intervention so far.

Accumulation of creatine in skeleton muscles in Post-COVID patients.

Tissue levels of creatine were measured with proton magnetic resonance spectroscopy (1.5 T Avanto scanner, Siemens, Erlangen, Germany) using matrix head coil in circularly polarized mode, with metabolite spectra in the specific regions of skeletal muscle and brain (vastus medials muscle, thalamus, frontal, precentral, paracentral, and parietal white and grey matter) processed as previously described (Appl Physiol Nutr Metab. 2016 Sep, 41(9):1005-7.).

### Statistical methods:

Initially, data were analyzed with the Shapiro-Wilk test for the normality of distribution and Bartlett's test for the homogeneity of the variances. When homogenous variances were verified for normally distributed data, summary measures for interaction effect (time vs. intervention) were compared by two-way ANOVA with repeated measures. When non-homogenous variances were identified, data were compared using Friedmann's test. Post-hoc LSD test and Wilcoxon test were used to identify differences between individual sample pairs for 2-way ANOVA and Friedmann's test, respectively. The significance level was set at P ≤ 0.05. Effect sizes after the intervention were assessed by Cohen statistics, with d ≥ 0.8 indicating a large effect. The data were analyzed using the statistical package SPSS version 24.0 for Mac (IBM SPSS Statistics, Chicago, IL).

Changes in tissue creatine levels in patients suffering under Post-COVID are summarized in Table 1. The Table shows changes in tissue creatine levels in white matter (brain), thalamus, vastus medialis and grey matter (brain) after intervention with 4 gram creatine per day for 3 months and 6 months compared to creatine level of the placebo group.

**Table. 1. Changes in tissue creatine level (mM)**

| | Creatine | | | Placebo | | |
|---|---|---|---|---|---|---|
| Total creatine (mM) | Baseline | 3 months | 6-months | Baseline | 3 months | 6-months |
| Vastus medialis muscle | 28.2 ± 3.0 | 30.0 ± 4.2 | 30.6 ± 3.4 | 20.6 ± 1.9 | 21.1 ± 3.1 | 21.9 ± 4.4 |
| Thalamus | 5.7 ± 0.5 | 6.3 ± 0.9 | 6.3 ± 1.0 | 5.9 ± 1.5 | 6.1 ± 0.8 | 6.0 ± 1.0 |
| Left frontal white matter | 5.9 ± 0.6 | 6.2 ± 0.6 | 6.2 ± 0.5 | 5.8 ± 0.6 | 5.7 ± 0.6 | 5.7 ± 0.4 |
| Right frontal white matter | 5.6 ± 0.8 | 6.6 ± 0.8 | 6.8 ± 1.0 | 5.7 ± 0.8 | 5.6 ± 0.7 | 5.6 ± 0.4 |
| Left frontal grey matter | 6.6 ± 0.4 | 6.9 ± 0.7 | 6.8 ±0.7 | 6.2 ± 0.7 | 6.2 ± 1.1 | 6.2 ± 0.8 |
| Right frontal grey matter | 6.5 ± 0.7 | 6.2 ± 0.3 | 6.2 ± 0.5 | 6.4 ± 0.7 | 6.3 ± 0.7 | 6.3 ± 0.6 |
| Left precentral white matter | 6.0 ± 0.6 | 6.2 ± 0.8 | 6.1 ± 0.7 | 5.8 ± 0.4 | 5.8 ± 0.3 | 5.9 ± 0.4 |
| Right precentral white matter | 5.8 ± 0.5 | 5.8 ± 0.7 | 6.0 ± 0.7 | 5.8 ± 0.3 | 5.8 ± 0.3 | 5.9 ± 0.2 |
| Left paracentral grey matter | 6.7 ± 0.6 | 7.3 ± 0.9 | 7.1 ± 0.7 | 6.5 ± 0.4 | 6.3 ± 0.6 | 6.4 ± 0.7 |
| Right paracentral grey matter | 6.8 ± 0.4 | 7.2 ± 0.5 | 7.0 ± 0.7 | 6.7 ± 0.4 | 6.8 ± 0.4 | 6.6 ± 0.4 |
| Left parietal white matter | 5.3 ± 0.6 | 6.0 ± 0.9 | 6.0 ± 0.6 | 5.2 ± 0.4 | 5.1 ± 0.4 | 5.0 ± 0.3 |
| Right parietal white matter | 5.3 ± 0.7 | 6.9 ± 0.9 | 6.8 ± 1.0 | 5.1 ± 0.6 | 5.2 ± 0.8 | 5.3 ± 0.5 |
| Left mesial grey matter | 7.2 ± 0.9 | 8.0 ± 1.2 | 8.0 ± 1.0 | 7.0 ± 0.7 | 7.0 ± 0.8 | 7.0 ± 0.9 |
| Right mesial grey matter | 7.1 ± 0.3 | 8.2 ± 1.3 | 8.1 ± 1.3 | 7.1 ± 0.4 | 7.2 ± 0.5 | 7.1 ± 0.3 |

The differences in percent of the creatine level after three months and the corresponding baseline level in white matter (brain), grey matter (brain), thalamus, and vastus medialis muscle calculated from Table 1 are shown in Figure 1. Left columns representing the group of patients receiving creatine, right columns representing the placebo group. For the values in white matter and grey matter average values are determined from Table 1.

In the randomized controlled trial, it was found that creatine is accumulated in brain and vastus medialis when administered for 3 months. The increased creatine level can be maintained for at least 3 further months by supplemental creatine administration. No changes in tissue creatine values were found in the placebo group.

The participants from the experimental group experienced an increase in tissue total creatine levels for all fourteen (14) locations evaluated in the study, with high increase at 6-month follow-up found in vastus medialis muscle (P = < 0.01), left frontal white matter (P = 0.01) and right parietal white matter (P = 0.01). No changes in tissue creatine values were found in the placebo group throughout the trial. Two-way ANOVA with repeated measures revealed a significant difference (treatment vs. time interaction) between interventions in tissue creatine levels (P < 0.05), with the creatine group superior to placebo to augment creatine levels at vastus medialis muscle, left frontal white matter, and right parietal white 6 matter. In addition, a strong interaction effect between interventions was reported for several other locations, including right frontal white matter, right paracentral grey matter, left parietal white matter, and left parietal mesial grey matter (P < 0.20).

In addition, the Cohen's effect sizes for primary and secondary outcomes after creatine intake with strong effect sizes of creatine (d ≥ 0.8) are demonstrated for increased brain levels in thalamus (0.82 at 3-month), right frontal white matter (1.25 at 3-month and 1.32 at 6-month), right paracentral grey matter (0.88 at 3-month), left parietal white matter (0.92 at 3-month and 1.17 at 6-month), parietal white matter (1.99 at 3-month and 1.74 at 6-month), left parietal messial grey matter (0.84 at 6-month), and right parietal messial grey matter (1.17 at 3-month and 1.06 at 6-month).

In sum, it was found that creatine is enriched in the brain of Post-COVID patients after supplementation with creatine and, thus, passes the blood-brain barrier.

Effect of creatine as nutrition supplement for Post-COVID patients.

The patient-reported outcomes for COVID-19-related signs and symptoms (e.g., breathing difficulties, lung pain, body aches,) were evaluated with VAS scale Table 3). Fatigue, comprising the conditions of the group general fatigue, physical fatigue, metal fatigue, reduced activity, and reduced motivation, was evaluated with a multidimensional fatigue inventory test (MFI-20 test, Smets E.M. et al., J. Psychosom. Res. 1995, 39(3), 315). The results of the VAS scale survey and of the MFI-20 test are summarized in Table 2.

**Table 2:**

| | Creatine | | | Placebo | | |
|---|---|---|---|---|---|---|
| | Baseline | 3 months | 6 months | Baseline | 3 months | 6 months |
| General fatigue | 12.0 | 11.3 | 12.7 | 12.0 | 11.8 | 12.8 |
| Physical fatigue | 13.5 | 12.7 | 13.2 | 12.5 | 12.7 | 12.8 |
| Mental fatigue | 12.3 | 11.2 | 10.5 | 11.0 | 11.3 | 12.2 |
| Reduced activity | 11.5 | 12.0 | 12.5 | 12.7 | 12.8 | 14.0 |
| Reduced motivation | 13.0 | 13.0 | 12.5 | 12.2 | 13.0 | 13.7 |

In the test performed, patients should report the score of the symptoms given in Table 2. Thus, the higher the score, the higher the respective symptom is experienced by the interviewed patient. As a consequence, the lower the value given, the better is the effect in mitigation of the respective symptom. As can be seen from Table 2, an improvement concerning physical fatigue is observed in the creatine group, which was not observed in the placebo group.

**Table. 3:**

| | Creatine | | | Placebo | | |
|---|---|---|---|---|---|---|
| | Baseline | 3 months | 6-months | Baseline | 3 months | 6-months |
| Breathing difficulties | 2.3 | 0.5 | 0.0 | 2.9 | 1.3 | 0.0 |
| Lung pain | 2.3 | 0.5 | 0.0 | 2.0 | 0.2 | 0.0 |
| Body Aches | 4.5 | 1.2 | 0.0 | 4.2 | 1.3 | 0.7 |

After 3 months breathing difficulties (dyspnea) are significantly reduced for 78% after creatine intake as compared to 55% after placebo intake. Body aches is reduced slightly for 73.3% after creatine intake as compared to 69% after placebo intake. Lung pain is reduced significantly in the creatine cohort.

A significant interaction effect (time vs. treatment) was found for all symptoms evaluated (P < 0.05). The Cohen's effect sizes for primary and secondary outcomes after creatine intake with strong effect sizes of creatine (d ≥ 0.8) are demonstrated also for breathing difficulties (0.89 at 3-month and 1.25 at 6-month), lung pain (0.99 at 6-month) and body aches (1.77 at 3-month and 3.03 at 6-month).

In the randomized controlled trial, it was found that creatine is accumulated in skeletal muscle and brain when administered for 3 months. The increase in endurance in the creatine group outcompetes placebo group (Table 5) . The improved endurance is associated with a reduction in body aches and lung pain and in particular with the recovery from breathing difficulties. The improved endurance is further associated with a reduction in fatigue symptoms, particularly with reduced mental fatigue and reduced motivation compared to an increase in the placebo group. The reduced motivation worsens over the observation period in the placebo group compared to slight improvement in the creatine cohort. Smaller improvements are achieved by creatine administration compared to the placebo group relating to fatigue symptoms like physical fatigue, and reduced activity according to the results presented in Table 2. In addition, creatine induced no major side effects.

### Example 2:

### Combination of creatine supplementation and pulmonary rehabilitation:

A second study was conducted to evaluate effectiveness and safety of replenishing creatine via dietary supplementation in patients suffering under Post-COVID after a SARS-CoV-2 infection. Eight male and female Post-COVID patients (age 33.5 ± 9.9 years, weight 72.3 ± 14.5 kg, and height 168.6 ± 11.0 cm; 4 women) with moderate fatigue and breathing difficulties or lung pain, lung malaise volunteered to participate in this randomized controlled trial. All patients were allocated in a double-blind parallel-group design to receive either 4 grams of creatine monohydrate per day plus breathing exercise (2-3 times per day for 10-15 min) (experimental group) or breathing exercise only (control group) during a 3-month intervention period.

Pulmonary rehabilitation is performed in accordance with Wang T.J. et al., Am J Phys Med Rehabil, 2020, Jun 11 (DOI 10.1097/PHM.0000000000001505 / PMCID: 7315835). Pulmonary rehabilitation is tailored to each individual patient and includes for example modified segmental breathing, breathing exercises for strengthening respiratory muscles and muscles of exhalation, inspiratory muscle training, bed mobility exercise, stretching, gymnastics, and/or walking. The extent of the exercises is low at the beginning and is slowly increased without exposing the patient higher efforts.

Detailed information on experimental protocols and testing procedures used are as described in Example 1.

All volunteers completed the trial, with no participants reported any side effects of either intervention. The changes in study outcomes during the trial are depicted in Table 5 and 6. The increase in tissue total creatine levels in several brain locations and in vastus medialis muscle are provided in Table 4.

**Table 4: Changes in tissue creatine level (mM)**

| | Creatine + Breathing Exercise | | Breathing Exercise | |
|---|---|---|---|---|
| Total creatine (mM) | Baseline | 3 months | Baseline | 3 months |
| Vastus medialis muscle | 30.5 ± 3.5 | 34.4 ± 6.3 | 30.1 ± 12.7 | 28.5 ± 9.2 |
| Thalamus | 5.0 ± 1.1 | 5.9 ± 0.9 | 5.5 ± 0.1 | 5.5 ± 0.1 |
| Left frontal white matter | 7.1 ± 1.2 | 8.2 ± 1.3 | 6.8 ± 0.2 | 6.7 ± 0.9 |
| Right frontal white matter | 6.1 ± 1.5 | 6.8 ± 0.9 | 6.5 ± 2.4 | 5.6 ± 0.8 |
| Left frontal grey matter | 6.6 ± 0.7 | 7.3 ± 1.1 | 6.5 ± 0.3 | 5.7 ± 0.4 |
| Right frontal grey matter | 6.6 ± 1.1 | 8.1 ± 0.5 | 7.9 ± 1.3 | 6.7 ± 1.0 |
| Left precentral white matter | 6.4 ± 0.5 | 6.6 ± 0.5 | 6.2 ± 0.1 | 6.4 ± 0.5 |
| Right precentral white matter | 6.1 ± 1.1 | 7.0 ± 1.3 | 5.0 ± 0.5 | 5.0 ± 0.5 |
| Left paracentral grey matter | 7.1 ± 0.9 | 8.5 ± 1.0 | 6.9 ± 0.1 | 5.7 ± 0.8 |
| Right paracentral grey matter | 7.2 ± 1.2 | 8.5 ± 0.4 | 6.8 ± 1.6 | 6.0 ± 1.6 |
| Left parietal white matter | 5.9 ± 2.1 | 6.7 ± 1.7 | 5.1 ± 0.8 | 5.1 ± 0.9 |
| Right parietal white matter | 5.5 ± 1.1 | 5.7 ± 0.9 | 5.4 ± 0.7 | 4.6 ± 0.1 |
| Left mesial grey matter | 8.0 ± 1.8 | 9.8 ± 2.5 | 7.3 ± 0.2 | 6.5 ± 0.1 |
| Right mesial grey matter | 8.2 ± 0.9 | 9.7 ± 1.5 | 6.0 ± 0.4 | 5.9 ± 0.2 |

**Table 5:**

| | Creatine + Breathing Exercise | | Breathing Exercise | |
|---|---|---|---|---|
| | Baseline | 3 months | Baseline | 3 months |
| General fatigue | 11.5 | 12.3 | 11.8 | 12.8 |
| Physical fatigue | 12.3 | 12.2 | 13.3 | 14.5 |
| Mental fatigue | 11.8 | 11.5 | 11.8 | 11.5 |
| Reduced activity | 12.5 | 12.8 | 12.5 | 12.8 |
| Reduced motivation | 13.5 | 12.8 | 16.0 | 13.0 |

**Table. 6**

| | Creatine + Breathing Exercise | | Breathing Exercise | |
|---|---|---|---|---|
| | Baseline | 3 months | Baseline | 3 months |
| Breathing difficulties | 1.5 | 0.0 | 2.0 | 0.0 |
| Lung pain | 0.5 | 0.0 | 2.8 | 0.0 |
| Lung malaise | 6.3 | 1.0 | 6.8 | 1.8 |

The participants from the creatine group experienced an increase in tissue total creatine levels for all fourteen (14) locations evaluated in our study, with significant increase at 3-month follow-up found in vastus medialis muscle (P = 0.04), thalamus (P = 0.03), right frontal grey matter (P = 0.04), right precentral white matter (P = 0.01), right paracentral grey matter (P = 0.03), and left parietal mesial grey matter (P = 0.01). The Cohen's effect sizes (d) for creatine increment at above six locations varied from 0.77 (vastus medialis muscle) to 1.76 (right frontal grey matter), implying a rather large effect of creatine monohydrate plus breathing exercise for muscle and brain creatine amplification. No elevation in total creatine levels was found for any location in the control group (except for a non-significant increase in left precentral white matter); furthermore, creatine levels significantly dropped in right frontal grey matter and left parietal mesial grey matter at 3-month follow-up in the control group (P < 0.05). In addition, two-way ANOVA with repeated measures revealed significant differences for changes in total creatine levels between groups at four brain locations (P < 0.05), with the participants from the experimental group were superior than the participants from the control group to augment brain creatine concentrations at left frontal grey matter, right frontal grey matter, right precentral white matter, and left parietal mesial grey matter.

Tissue total creatine concentrations at 3-month follow-up remained largely unresponsive to breathing exercise (or even further diminished from baseline levels), suggesting long-term disturbances in tissue bioenergetics after this complex condition.

Breathing difficulties and lung pain were reduced to zero in both groups at 3-month follow up, while lung malaise was significantly reduced for 84% in the creatine group, and for 74% in the control group (P < 0.05). Friedman / ANOVA test with repeated measures revealed significant differences for lung malaise between the groups (P = 0.03), with the participants from the creatine group were superior than the participants from the control group for lung malaise reduction. The Cohen's effect size for lung malaise was 2.97, suggesting a large effect of creatine monohydrate plus breathing exercise (Table 6). The outcome is confirmed by the results of the VAS scale survey concerning physical fatigue. Particularly physical fatigue is significantly improved by administration of creatine in combination with breathing exercise (Table 5). No significant improved on mental fatigue and reduced motivation is achieved by creatine + breathing exercise compared to the control group (breathing exercise group).

### Example 3: Exhaustion studies

The time to exhaustion of the patients was evaluated by an incremental test until exhaustion on a motorized treadmill. Speed and gradient of the treadmill are increased every third minute, starting at 2.7 km/h (1.7 miles per hour) at a 10% gradient and rising at stage 7 to 9.7 km/h (6 miles per hour) at 22% gradient (Will P.M. and Walter J.D., Am Heart. J., 1999 Dec, 138, 1033). The results of the treadmill evaluation are provided in Table 7 and 8.

**Table 7:**

| | Creatine | | | Placebo | | |
|---|---|---|---|---|---|---|
| | Baseline | 3-months | 6-months | Baseline | 3-months | 6-months |
| Time to exhaustion (sec) | 894 | 922 | 959 | 879 | 883 | 897 |

The time to exhaustion is significantly increased in patients suffering under post viral fatigue syndrome after a SARS-CoV-2 virus infection. The time to exhaustion of the creatine group is increased 7.3% compared to 2.1% after placebo intake. More important, the creatine group achieves stage 6 in the treadmill test, wherein the placebo group remains at stage 5.

An increase in time to exhaustion in creatine group, with creatine outcompetes placebo in prolonging time to exhaustion after 3-months and 6-months administration of creatine. The improved endurance is associated with a reduction in body aches and lung pain and in particular with the recovery from breathing difficulties.

**Table 8:**

| | Creatine + Breathing Exercise | | | Breathing Exercise | | |
|---|---|---|---|---|---|---|
| | Baseline | 3-months | 6-months | Baseline | 3-months | 6-months |
| Time to exhaustion (sec) | 867 | 921 | -- | 807 | 840 | -- |

The mean time to exhaustion was significantly improved for 54 sec in the experimental group (P = 0.05) at the post-administration, with creatine monohydrate plus breathing exercise, is superior to breathing exercise to extend time to exhaustion (P = 0.11). The Cohen's effect size for time to exhaustion was 0.36. The creatine + breathing exercise group reaches stage 6 in the treadmill test, wherein the placebo group remains at stage 5. The data illustrates a synergistic effect of creatine monohydrate plus breathing exercise compared to the placebo group.

### Conclusions:

Creatine monohydrate plus breathing exercise can be recommended as a well-tolerated intervention to notably improve tissue creatine levels and several clinical features (including breathing difficulties, lung pain, lung malaise and time to exhaustion) in patients with Post-COVID fatigue syndrome when administered during a 3-month treatment period, for example.

### Example 4:

### Creatine supplementation compared to glucose administration:

Ten male and female PCFS patients with moderate fatigue and at least one of additional COVID-related symptoms (e.g. ageusia, anosmia, body aches, breathing difficulties, difficulties concentrating, headache, lung pain, malaise) volunteered to participate in this randomized controlled parallel-group interventional trial. All patients were allocated in a double-blind parallel-group design to receive a powder creatine monohydrate (experimental group; 8 grams of Creatine (Creapure^{®}) per day or glucose (control group; 3 grams of glucose per day) t.i.d. during an 8-week intervention interval. All participants refrained from using any other dietary supplements during the study. Detailed information about experimental protocols and testing procedures used in this study are described in Example 1.

Changes in tissue creatine levels in patients suffering under Post-COVID are depicted in Table 9. The Table shows changes in tissue creatine levels in white matter (brain), thalamus, vastus medialis and grey matter (brain) after creatine intervention compared to the glucose group.

**Table 9: Changes in tissue creatine level (mM)**

| | Creatine | | Glucose | |
|---|---|---|---|---|
| Total creatine (mM) | Basline | 8 weeks | Baseline | 8 weeks |
| Vastus medialis muscle | 29.0 ± 5.6 | 30.4 ± 5.2 | 30.1 ± 12.7 | 28.5 ± 9.2 |
| Thalamus | 5.1 ± 1,0 | 5.3 ± 0.9 | 5.5 ± 0.5 | 5.5 ± 0.7 |
| Left frontal white matter | 6.7 ± 0.7 | 6.8 ± 0.9 | 5.8 ± 0.4 | 5.6 ± 1.0 |
| Right frontal white matter | 6.7 ± 1.0 | 7.0 ± 1.1 | 5.6 ± 0.8 | 5.6 ± 0.8 |
| Left frontal grey matter | 6.6 ± 0.6 | 6.5 ± 0.5 | 6.8 ± 0.4 | 6.3 ± 0.9 |
| Right frontal grey matter | 6.6 ± 1.1 | 6.7 ± 1.0 | 7.0 ± 0.9 | 7.0 ± 0.7 |
| Left precentral white matter | 6.4 ± 1.0 | 6.7 ± 0.8 | 5.6 ± 0.6 | 5.6 ± 0.5 |
| Right precentral white matter | 6.8 ± 0.9 | 7.4 ± 1.1 | 5.4 ± 0.6 | 5.3 ± 0.7 |
| Left paracentral grey matter | 6.8 ± 0.8 | 6.9 ± 0.9 | 7.0 ± 0.9 | 6.9 ± 0.7 |
| Right paracentral grey matter | 7.2 ± 0.8 | 7.6 ± 1.6 | 6.4 ± 0.7 | 6.2 ± 1.1 |
| Left parietal white matter | 7.5 ± 1.6 | 7.8 ± 1.3 | 5.4 ± 0.6 | 5.3 ± 0.5 |
| Right parietal white matter | 7.6 ± 1.9 | 7.7 ± 1.9 | 5.2 ± 0.7 | 5.2 ± 0.7 |
| Left mesial grey matter | 8.1 ± 1.3 | 8.6 ± 1.1 | 7.6 ± 0.7 | 7.4 ± 0.6 |
| Right mesial grey matter | 8.4 ± 1.4 | 9.2 ± 1.0 | 7.3 ± 0.6 | 7.0 ± 1.0 |

Creatine levels after 8 weeks in white matter (brain), grey matter (brain), thalamus, and vastus medialis muscle are increased in the experimental group compared with the control group. The results confirms the outcomes of examples 1 (Table 1) and 2 (Table 4).

The patient-reported outcomes for COVID-19-related signs and symptoms (e.g., breathing difficulties, lung pain, body aches and post viral fatigue) were evaluated with VAS scale (Tables 10 and 11).

**Table 10:**

| | Creatine | | Gluose | |
|---|---|---|---|---|
| | Baseline | 8 weeks | Baseline | 8 weeks |
| General fatigue | 12.6 | 12.0 | 12.0 | 11.6 |
| Physical fatigue | 12.2 | 11.4 | 12.8 | 12.6 |
| Mental fatigue | 13.4 | 12.2 | 11.4 | 11.0 |
| Reduced activity | 13.6 | 12.0 | 12.6 | 12.6 |
| Reduced motivation | 11.6 | 10.3 | 10.0 | 10.8 |

**Table 11:**

| | Creatine | | Glucose | |
|---|---|---|---|---|
| | Baseline | 8 weeks | Baseline | 8 weeks |
| Breathing difficulties | 2.6 | 0.4 | 1.4 | 0.8 |
| Lung pain | 1.8 | 0.6 | 0.4 | 0.6 |
| Body aches | 2.8 | 0.8 | 1.2 | 0.6 |

After 8 weeks breathing difficulties (dyspnea) are significantly reduced for 85% after creatine intake as compared to 43% after glucose intake. Body aches is reduced for 71% after creatine intake as compared to 50% after glucose intake. Lung pain is reduced significantly for 67% in the creatine cohort as compared to an increase of 50% in the glucose cohort.

Thus, several Post-COVID-related symptoms such as body aches, lung pain and breathing difficulties were significantly reduced in the experimental group (creatine) at 8-week follow-up (P ≤ 0.05); the Cohen's effect sizes (d) for reducing breathing difficulties, body aches and lung malaise were above a threshold value of 0.80, suggesting a large effect of creatine. The participants from the experimental group (creatine) are superior to the participants from control group (glucose) to attenuate body aches and lung pain (P ≤ 0.20), the participants from the experimental group are superior to the participants of the control group to attenuate breathing difficulties, also.

The Cohen's effect sizes (d) in the creatine group for all outcomes evaluated and reproduced in Table 12 and Figure 2. The effects are classified as small (d = 0.2), medium (d = 0.5, dashed line), and large (d ≥ 0.8). Missing values are due to a statistical limitation in calculating effect sizes when pre and/or post values equal zero. Abbreviations: GF, general fatigue; PF, physical fatigue; RM, reduced motivation; RA, reduced activity; MF, mental fatigue; BOA, body aches; BRD, breathing difficulties; LUP, lung pain; and MAL, malaise.

**Table 12:**

| | Cohen's effect (d) |
|---|---|
| General Fatigue (GE) | 0,44 |
| Physical Fatigue (PF) | 0,83 |
| Reduced Motivation (RM) | 1,07 |
| Reduced activity (RA) | 1,52 |
| Mental Fatigue (MF) | 1,32 |
| Breathing Difficulties (BRD) | 0,96 |
| Body aches (BOA) | 1,11 |
| Lung pain (LUP) | 0,64 |
| Malaise (MAL) | 1,81 |

## Claims

1. Pharmaceutical composition comprising creatine or a creatine hydrate and/or a salt thereof for use in the treatment of one of the conditions selected from group of physical fatigue and breathing difficulties and/or to increase time to exhaustion, after a SARS-CoV-2 infection.

2. The pharmaceutical composition for use of claim 1, wherein the physical fatigue and/or breathing difficulties are caused by a SARS-CoV-2 infection or are symptoms of Post-COVID after a SARS-CoV-2 infection.

3. The pharmaceutical composition of claim 1 or 2 for use in the treatment of breathing difficulties and/or physical fatigue in combination with physical exercises and/or breathing exercises.

4. The pharmaceutical composition of any one of claims 1 to 3 for use in the treatment of physical fatigue in subjects undergoing physical exercises and/or breathing exercises.

5. The pharmaceutical composition for use of any one of claims 1 to 4, wherein the composition is for use in combination with breathing exercises for strengthening respiratory muscles including strengthening of the diaphragm, the external intercostal muscles and the muscles of exhalation.

6. The pharmaceutical composition for use of any one of claims 1 to 5, wherein the composition is for use in combination with breathing exercises and wherein the breathing exercises are gradually increased as tolerated by the subject in need thereof.

7. The pharmaceutical composition for use of any one of claims 1 to 6, wherein the creatine salt is selected from the group consisting of the corresponding acetates, citrates, maleates, fumarates, tartrates, malates, pyruvates, ascorbates, succinates, aspartates, lactates, oxalates, formates, benzoates, phosphates, sulfates, chlorides, hydrochlorides, of the corresponding potassium, sodium, calcium, magnesium salts.

8. The pharmaceutical composition for use of any one of claims 1 to 7, wherein the composition comprises creatine monohydrate.

9. The pharmaceutical composition for use of any one of claims 1 to 8, wherein the daily dose of creatine in the composition is in the range of 7g to 30g.

10. The pharmaceutical composition for use of any one of claims 1 to 8, wherein the daily dose of creatine in the composition is an accumulation dose in the range of 10g to 30g for an initial accumulation phase and a maintaining dose in the range of 7g to 15g for a subsequent maintaining phase, wherein the accumulation phase has a duration of up to 3 weeks and the maintaining phase has a duration of 1 month to 12 months.

11. A dietary supplement comprising creatine or a creatine hydrate and/or salt thereof for use in supporting the recovery from one of the conditions selected from the group of physical fatigue and breathing difficulties and/or to increase time to exhaustion, after a SARS-CoV-2 infection.

12. The dietary supplement for use of claim 11, wherein the physical fatigue and/or breathing difficulties are caused by a SARS-CoV-2 infection or are symptoms of Post-COVID after a SARS-CoV-2 infection.

13. The dietary supplement for use of claim 11 or 12, wherein the composition is used in combination with physical exercises and/or breathing exercises for strengthening respiratory muscles including strengthening of the diaphragm, the external intercostal muscles and the muscles of exhalation.

14. The dietary supplement for use of claims 11 to 13, wherein the daily dose of creatine in the composition is in the range of 7g to 30g.

15. The dietary supplement for use of claims 11 to 14, wherein the daily dose of creatine in the composition is an accumulation dose in the range of 10g to 30g for an initial accumulation phase and a maintaining dose in the range of 7g to 15g for a subsequent maintaining phase, wherein the accumulation phase has a duration of up to 3 weeks and the maintaining phase has a duration of 1 month to 12 months.

16. The dietary supplement for use of claims 11 to 15, wherein the used composition comprises creatine monohydrate.

## Patentansprüche

1. Pharmazeutische Zusammensetzung, die Kreatin oder ein Kreatinhydrat und/oder ein Salz davon umfasst, zur Verwendung bei der Behandlung einer der Erkrankungen, ausgewählt aus der Gruppe von körperlicher Erschöpfung und Atembeschwerden, und/oder zur Verlängerung der Zeit bis zur Erschöpfung nach einer SARS-CoV-2-Infektion.

2. Pharmazeutische Zusammensetzung zur Verwendung nach Anspruch 1, wobei die körperliche Erschöpfung und/oder Atembeschwerden durch eine SARS-CoV-2-Infektion verursacht werden oder Symptome von Post-COVID nach einer SARS-CoV-2-Infektion sind.

3. Pharmazeutische Zusammensetzung nach Anspruch 1 oder 2 zur Verwendung bei der Behandlung von Atembeschwerden und/oder körperlicher Erschöpfung in Kombination mit körperlichen Übungen und/oder Atemübungen.

4. Pharmazeutische Zusammensetzung nach einem der Ansprüche 1 bis 3 zur Verwendung bei der Behandlung von körperlicher Erschöpfung bei Patienten, die körperliche Übungen und/oder Atemübungen durchführen.

5. Pharmazeutische Zusammensetzung zur Verwendung nach einem der Ansprüche 1 bis 4, wobei die Zusammensetzung zur Verwendung in Kombination mit Atemübungen zur Stärkung der Atemmuskulatur, einschließlich der Stärkung des Zwerchfells, der äußeren Interkostalmuskeln und der Ausatmungsmuskeln, bestimmt ist.

6. Pharmazeutische Zusammensetzung zur Verwendung nach einem der Ansprüche 1 bis 5, wobei die Zusammensetzung zur Verwendung in Kombination mit Atemübungen bestimmt ist und wobei die Atemübungen schrittweise entsprechend der Verträglichkeit des Patienten, der sie benötigt, gesteigert werden.

7. Pharmazeutische Zusammensetzung zur Verwendung nach einem der Ansprüche 1 bis 6, wobei das Kreatinsalz aus der Gruppe ausgewählt ist, die aus den entsprechenden Acetaten, Citraten, Maleaten, Fumaten, Tartraten, Malaten, Pyruvaten, Ascorbaten, Succinaten, Aspartaten, Lactaten, Oxalaten, Formaten, Benzoaten, Phosphaten, Sulfaten, Chloriden, Hydrochloriden, oder entsprechenden Kalium-, Natrium-, Calcium-und Magnesiumsalzen besteht.

8. Pharmazeutische Zusammensetzung zur Verwendung nach einem der Ansprüche 1 bis 7, wobei die Zusammensetzung Kreatinmonohydrat umfasst.

9. Pharmazeutische Zusammensetzung zur Verwendung nach einem der Ansprüche 1 bis 8, wobei die Tagesdosis an Kreatin in der Zusammensetzung im Bereich von 7 g bis 30 g liegt.

10. Pharmazeutische Zusammensetzung zur Verwendung nach einem der Ansprüche 1 bis 8, wobei die Tagesdosis an Kreatin in der Zusammensetzung eine Akkumulationsdosis im Bereich von 10 g bis 30 g für eine anfängliche Akkumulationsphase und eine Erhaltungsdosis im Bereich von 7 g bis 15 g für eine anschließende Erhaltungsphase ist, wobei die Akkumulationsphase eine Dauer von bis zu 3 Wochen und die Erhaltungsphase eine Dauer von 1 Monat bis 12 Monaten hat.

11. Ein Nahrungsergänzungsmittel umfassend Kreatin oder ein Kreatinhydrat und/oder ein Salz davon zur Verwendung zur Unterstützung der Erholung von einem der Zustände, ausgewählt aus der Gruppe von körperlicher Erschöpfung und Atembeschwerden, und/oder zur Verlängerung der Zeit bis zur Erschöpfung nach einer SARS-CoV-2-Infektion.

12. Nahrungsergänzungsmittel zur Verwendung nach Anspruch 11, wobei die körperliche Erschöpfung und/oder die Atembeschwerden durch eine SARS-CoV-2-Infektion verursacht werden oder Symptome von Post-COVID nach einer SARS-CoV-2-Infektion sind.

13. Nahrungsergänzungsmittel zur Verwendung nach Anspruch 11 oder 12, wobei die Zusammensetzung in Kombination mit körperlichen Übungen und/oder Atemübungen zur Stärkung der Atemmuskulatur, einschließlich der Stärkung des Zwerchfells, der äußeren Interkostalmuskeln und der Ausatmungsmuskeln, verwendet wird.

14. Nahrungsergänzungsmittel zur Verwendung nach einem der Ansprüche 11 bis 13, wobei die Tagesdosis an Kreatin in der Zusammensetzung im Bereich von 7 g bis 30 g liegt.

15. Nahrungsergänzungsmittel zur Verwendung nach einem der Ansprüche 11 bis 14, wobei die Tagesdosis an Kreatin in der Zusammensetzung eine Akkumulationsdosis im Bereich von 10 g bis 30 g für eine anfängliche Akkumulationsphase und eine Erhaltungsdosis im Bereich von 7 g bis 15 g für eine anschließende Erhaltungsphase ist, wobei die Akkumulationsphase eine Dauer von bis zu 3 Wochen und die Erhaltungsphase eine Dauer von 1 Monat bis 12 Monaten hat.

16. Nahrungsergänzungsmittel zur Verwendung nach einem der Ansprüche 11 bis 15, wobei die verwendete Zusammensetzung Kreatinmonohydrat umfasst.

## Revendications

1. Composition pharmaceutique comprenant de la créatine ou un hydrate de créatine et/ou un sel de celle-ci, destinée à être utilisée dans le traitement de l'une des affections choisies dans le groupe de la fatigue physique et des difficultés respiratoires et/ou pour augmenter la durée jusqu'à l'épuisement, après une infection par le SARS-CoV-2.

2. Composition pharmaceutique destinée à être utilisée selon la revendication 1, où la fatigue physique et/ou les difficultés respiratoires sont causées par une infection par le SARS-CoV-2 ou sont des symptômes de post-COVID après une infection par le SARS-CoV-2.

3. Composition pharmaceutique selon la revendication 1 ou 2, destinée à être utilisée dans le traitement des difficultés respiratoires et/ou de la fatigue physique en association avec des exercices physiques et/ou des exercices respiratoires.

4. Composition pharmaceutique selon l'une quelconque des revendications 1 à 3, destinée à être utilisée dans le traitement de la fatigue physique chez les sujets subissant des exercices physiques et/ou des exercices respiratoires.

5. Composition pharmaceutique destinée à être utilisée selon l'une quelconque des revendications 1 à 4, où la composition est destinée à être utilisée en association avec des exercices respiratoires pour le renforcement des muscles respiratoires, y compris le renforcement du diaphragme, des muscles intercostaux externes et des muscles expiratoires.

6. Composition pharmaceutique destinée à être utilisée selon l'une quelconque des revendications 1 à 5, où la composition est destinée à être utilisée en association avec des exercices respiratoires et où les exercices respiratoires sont augmentés progressivement selon ce qui est toléré par le sujet qui en a besoin.

7. Composition pharmaceutique destinée à être utilisée selon l'une quelconque des revendications 1 à 6, où le sel de créatine est choisi dans le groupe consistant en les acétates, les citrates, les maléates, les fumarates, les tartrates, les malates, les pyruvates, les ascorbates, les succinates, les aspartates, les lactates, les oxalates, les formiates, les benzoates, les phosphates, les sulfates, les chlorures, les chlorhydrates correspondants, ou les sels de potassium, de sodium, de calcium, de magnésium correspondants.

8. Composition pharmaceutique destinée à être utilisée selon l'une quelconque des revendications 1 à 7, où la composition comprend du monohydrate de créatine.

9. Composition pharmaceutique destinée à être utilisée selon l'une quelconque des revendications 1 à 8, où la dose journalière de créatine dans la composition est dans la plage de 7 g à 30 g.

10. Composition pharmaceutique destinée à être utilisée selon l'une quelconque des revendications 1 à 8, où la dose journalière de créatine dans la composition est une dose d'accumulation dans la plage de 10 g à 30 g pour une phase d'accumulation initiale et une dose de maintien dans la plage de 7 g à 15 g pour une phase de maintien ultérieure, où la phase d'accumulation a une durée de jusqu'à 3 semaines et la phase de maintien a une durée de 1 mois à 12 mois.

11. Complément alimentaire comprenant de la créatine ou un hydrate de créatine et/ou un sel de celle-ci, destiné à être utilisé pour favoriser la récupération après l'une des affections choisies dans le groupe de la fatigue physique et des difficultés respiratoires, et/ou pour augmenter la durée jusqu'à l'épuisement, après une infection par le SARS-CoV-2.

12. Complément alimentaire destiné à être utilisé selon la revendication 11, où la fatigue physique et/ou les difficultés respiratoires sont causées par une infection par le SARS-CoV-2 ou sont des symptômes de post-COVID après une infection par le SARS-CoV-2.

13. Complément alimentaire destiné à être utilisé selon la revendication 11 ou 12, où la composition est utilisée en association avec des exercices physiques et/ou des exercices respiratoires pour le renforcement des muscles respiratoires, y compris le renforcement du diaphragme, des muscles intercostaux externes et des muscles expiratoires.

14. Complément alimentaire destiné à être utilisé selon les revendications 11 à 13, où la dose journalière de créatine dans la composition est dans la plage de 7 g à 30 g.

15. Complément alimentaire destiné à être utilisé selon les revendications 11 à 14, où la dose journalière de créatine dans la composition est une dose d'accumulation dans la plage de 10 g à 30 g pour une phase d'accumulation initiale et une dose de maintien dans la plage de 7 g à 15 g pour une phase de maintien ultérieure, où la phase d'accumulation a une durée de jusqu'à 3 semaines et la phase de maintien a une durée de 1 mois à 12 mois.

16. Complément alimentaire destiné à être utilisé selon les revendications 11 à 15, où la composition utilisée comprend du monohydrate de créatine.
